(19) Europäisches Patentamt
European Patent Office
Office européen des brevets

(11) Publication number: 0 190 609 B1

(12) EUROPEAN PATENT SPECIFICATION

(45) Date of publication of patent specification: 03.01.90

(21) Application number: 86100825.8

(22) Date of filing: 22.01.86

(51) Int. Cl.$^5$: C 07 D 301/12, C 07 D 303/04, C 07 D 303/16, C 07 D 303/22 // B01J29/04

# (54) Process for the monoepoxidation of compounds containing two double bonds.

(30) Priority: 05.02.85 IT 1939085

(43) Date of publication of application: 13.08.86 Bulletin 86/33

(45) Publication of the grant of the patent: 03.01.90 Bulletin 90/01

(84) Designated Contracting States: BE CH DE FR GB IT LI NL SE

(56) References cited:
EP-A-0 100 119
GB-A-1 143 433

(73) Proprietor: ENICHEM SYNTHESIS S.p.A.
Via Ruggero Settimo 55
I-90139 Palermo (IT)

(72) Inventor: Maspero, Federico
Via Jannozzi, 18
San Donato Milanese Milano (IT)
Inventor: Romano, Ugo
Via XXV Aprile, 10
Vimercate Milano (IT)

(74) Representative: Cioni, Carlo et al
c/o ENIRICERCHE S.p.A. BRELID Via F. Maritano 26
I-20097 San Donato Milanese (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 190 609 B1

Courier Press, Leamington Spa, England.

## Description

The present invention relates to a process for the monoepoxidation with $H_2O_2$ of compounds containing two olephinic double bonds.

More particularly, the present invention relates to a process for the monoepoxidation with $H_2O_2$ of compounds with two olephinic double bonds containing at least an allyl group, or of diolephinic compounds. It is known that hydrogen peroxide is capable of epoxidizing monoolephinically unsaturated compounds. As an example, GB—A—1,143,433 describes the epoxidation of unsaturated hydrocarbons containing at least one olefinic double bond in liquid phase utilising hydrogen peroxide in the presence of a cation exchange resin of the carboxylic type.

The process is known in particular as disclosed in the Italian Patent Application 22608 A/82, according to which the olephins can be epoxidized by means of hydrogen peroxide in the presence of a synthetic zeolite (titanium-silicalite) containing Ti atoms replacing silicon inside silica's crystalline lattice.

It is known that the epoxidation occurs in correspondence of the olephinic double bonds, so that compounds containing two double bonds were thought to yield di-epoxides on epoxidation.

It has been surprisingly found that compounds containing two double bonds can be transformed into mono-epoxides by hydrogen peroxide, by using the titanium-silicalite of the above-mentioned Italian Patent Application, when inside their molecules the double bonds are separated by less than 8 carbon atoms, or equivalent groups (—O—, —N—, —$SO_2$—, and the like).

The process being the object of the present invention is particularly valuable for compounds with two double bonds containing at least an allyl group, or for diolephinic compounds with a number of carbon atoms not lower than four, in particular butadiene-1,3.

The process being the object of the present invention comprises placing the compound with two double bonds, with the exclusion of isoprene, in contact with hydrogen peroxide in aqueous solution at a concentration of from 3% to 70% by weight, in the presence of a catalyst constituted by titanium-silicalite (Si-Ti synthetic zeolite, wherein Ti and Si in the form of their oxides $TiO_2$ and $SiO_2$ have a molar ratio in the zeolite corresponding to the formula

$$xTiO_2 \cdot (1-x)SiO_2$$

with x being comprised within the range of from 0.0001 to 0.04, preferably of from 0.0055 to 0.036), possibly in the presence of at least one solvent of polar nature, at such a temperature and under such a pressure as to keep the whole reaction mass in the liquid phase.

As for the solvents which can be used according to the present invention, they are in particular alcohols, ketones, ethers, glycol-ethers, glycols, with a number of carbon atoms lower than or equal to 6.

More preferably, among alcohols methanol or tert.-butanol; among ketones, acetone; and among ethers, (2)-methoxy-(1)-propanol can be used.

As for the titanium-silicalite to be used as the catalyst according to the invention, it is in particular disclosed in Belgian Patent N. 886812.

We report hereinunder the process for the preparation of titanium-silicalite, and the properties thereof.

The process for the preparation of titanium-silicalite comprises the preparation of a reaction mixture free or substantially free from alkaline metals, constituted by sources of silicon oxide, and titanium oxide, a nitrogen-containing organic base and water, having a composition, as expressed in terms of reactants' molar ratios, defined as follows:

| Reactants | Molar ratios | Preferably |
|---|---|---|
| $SiO_2/TiO_2$ | 5—200 | 35—65 |
| $OH/SiO_2$ | 0.1—1.0 | 0.3—0.6 |
| $H_2O/SiO_2$ | 20—200 | 60—100 |
| $RN^+/SiO_2$ | 0.1—2.0 | 0.4—1.0 |

By $RN^+$, we have indicated the nitrogen-containing organic cation deriving from the organic base used for the preparation of titanium-silicalite (TS-1).

The source of silicon oxide can be a tetraalkylorthosilicate, preferably tetraethyl - orthosilicate, or also simply a silica in colloidal form.

The source of titanium oxide is a hydrolizable titanium compound preferably selected among $TiCl_4$, $TiOCl_2$, Ti - (alkoxy)$_4$, preferably Ti $(OC_2H_5)_4$.

The organic base is tetraalkyl-ammonium hydroxide, in particular tetrapropyl - ammonium hydroxide.

The mixture of reactants undergoes a hydrothermal treatment in autoclave at temperatures comprised within the range of from 130 to 200°C, under the autogenous pressure, and for a time of from 6 to 30 days,

until the crystals of titanium-silicalite (TS-1) precursor have been formed; such crystals are separated from mother solution, thoroughly washed with water and dried; in the anhydrous state, they have the following composition:

$$xTiO_2 \cdot (1\text{-}x)SiO_2 \cdot 0.04(RN^+)_2O.$$

Precursor's crystals are heated for from 1 to 72 hours in air, at 550°C, to the purpose of completely removing the nitrogen-containing organic base. End TS-1 has the composition:

$$xTiO_2 \cdot (1\text{-}x)SiO_2$$

with x being as hereinabove defined, i.e., ranging from 0.0001 to 0.04, preferably from 0.0055 to 0.036.

TS-1 can contain alkaline metals in concentrations of up to 50 ppm, and possibily iron up to 30 ppm.

TS-1 is of silicalite type, and all titanium replaces silicon. The synthetic material shows characteristics which are evidenced by X-ray and I.R. analyses.

X-Ray analysis is carried out by diffractometer for powders, provided with electronic system for impulse counting, using CuK radiation.

Titanium-silicalites (TS-1) are characterized by an X-ray diffraction spectrum, as shown in Figure 1b; such spectrum is roughly similar to silicalite's typical spectrum (Figure 1a); it shows however some clearly "singlet" reflections wherein, in pure silicalite's spectrum doublet reflections are evident.

As the differences in TS-1 and silicalite spectra are relatively minor, a particular precision is required in spectrum detection; for such a reason, TS-1 and silicalite have been examined with the same equipment, using as well $Al_2O_3$ as internal standard.

In Table 1 the most significant data are reported from spectra of a TS-1 with x=0.017, and of a pure silicalite.

The constants of lattice's elemental cell have been determined by means of the method of minimum squares, on the basis of the values of interplanar distances of 7—8 single reflections comprised within the range of 10—40° 2θ.

A large number of the values of TS-1 interplanar distances trend to be, even if slightly, higher than corresponding ones of pure silicalite, according to the higher values foreseeable for the Ti-O bond length, than for Si-O bond.

The turning from a doublet to a single reflection is interpreted as a turning from a monoclinic (pseudo-orthorhombic) symmetry (silicalite) to a real orthorhombic symmetry, "titanium-silicalite" (TS-1). In Figure 1, the most evident spectrum differences as above are indicated by arrows.

Infrared analysis

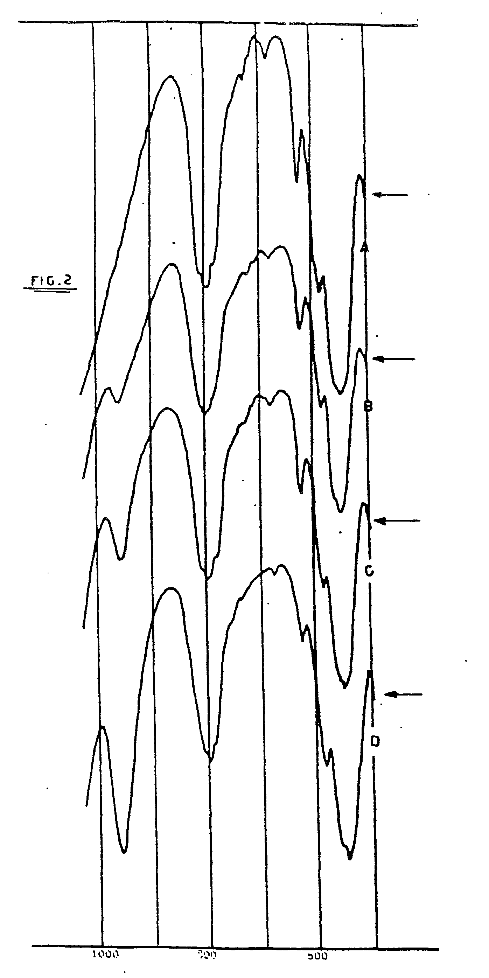

TS-1 shows a characteristic absorption band at about 950 $cm^{-1}$ (see Figure 2, spectra B, C and D), which is not present in the spectrum of pure silicalite (Figure 2, spectrum A), as well as it is absent from spectra of titanium oxides (rutile, anatase) and of alkaline titanates.

Spectrum B is the spectrum of TS-1 with 5% of $TiO_2$ by mole; spectrum C is that of TS-1 with 8% of $TiO_2$ by mole; and spectrum D is finally that of TS-1 with 23% of $TiO_2$ by mole.

As it can be seen in Figure 2, the intensity of the band at about 950 $cm^{-1}$ increases with increasing content of silicon-replacing titanium in silicalite structure.

Morphology

From a morphological point of view, TS-1 appears as parallelepipedons with bevelled corners. An examination carried out by X-ray microprobe has demonstrated that the distribution of titanium inside the crystal is perfectly uniform, thus confirming that titanium is replacing silicon inside the silicalite lattice structure, and is not present in other forms.

As for the reaction conditions, it must be observed that an excess of compound with two double bonds in reaction medium can be used when said compound can be easily distilled off under vacuum at low enough temperature.

In this way, the reaction proceeds up to the desired conversion and the excess can be easily removed without excessively heating the mixture containing the epoxidized derivative, which is usually heat-sensible.

On the contrary, when said method cannot be practiced, in that the compound cannot be easily distilled in vacuo at low enough temperature, an excess is used of $H_2O_2$, and this latter is removed by water-washing. After this washing step, removing the solvent in vacuo, without distilling the product is enough. As for the catalyst, it is removed at the end of reaction by filtration or centrifuging.

The temperature and the pressure used during the reaction are such as to maintain the whole mass in the liquid phase, and are respectively comprised within the ranges of from 0°C to 150°C, and of from 0.10 to 20.26 bar.

We are now supplying Examples to the purpose of better illustrating the invention, it being to be understood that the invention is not to be considered as being limited to them or by them.

Example 1
Allyl glycidyl carbonate (AGC)

To 50 g of diallylcarbonate (DAC) (0.35 mol) in 20 cc of methanol, plus 1,5 g of titanium-silicalite (%TiO=4.0; Fe=6 ppm; Na=42 ppm; K=3 ppm), 6 cc of 60% $H_2O_2$ (0.14 mol) are added, and the mixture is stirred for 6 hours at 20°C. The reaction mixture is centrifuged to the purpose of removing the catalyst, and the solvent and excess DAC are distilled off in vacuo (45°C under 13.3 Pa), a residue (23 g) containing 93% of AGC (the balance being constituted by diglycidylcarbonate), peroxide-free, being obtained. If obtaining a more pure product is desired, a distillation in vacuo (85—90°C under 13.3 Pa) can be carried out, with a yield of 20 g of pure AGC.

Example 2
Glycidyl acrylate

To 40 cc of allyl acrylate (0.35 mol) in 200 cc of acetone, plus titanium-silicalite (5 g) (the same as of Example 1), 20 ml of 60% $H_2O_2$ (0.44 mol) are added.

After a 15-hour stirring at 20°C, a centrifuging is carried out to remove the catalyst, then 200 cc each of water and toluene are added to extract the organic portion.

The aqueous phase is washed with further 100 cc of toluene. To the combined organic extracts 1 mg is added of hydroquinone monomethyl ether as stabilizer, and solvent and unconverted allyl acrylate are distilled off in vacuo at room temperature.

A residue is obtained of 33.1 g of glycidyl acrylate, pure enough not to be distilled.

Example 3
Glycidyl metacrylate

The same process as used for glycidyl acrylate is used, starting from allyl metacrylate (45 g). Thirty-five grams of not-distilled glycidyl metacrylate are obtained.

Example 4
Butadiene monoepoxide

Into a 250-cc flask, 50 g of molten tert.butyl alcohol plus 1 g of titanium-silicalite (%$TiO_2$=2.80; Na=9 ppm; K=2 ppm; Fe=30 ppm) are placed. Butadiene is bubbled at the rate of 1.5 l/h, under stirring, and contemporaneously 5 cc of 60% $H_2O_2$ (0.11 mol), diluted in 10 cc of tert.butyl alcohol, are added dropwise over a 1-hour time, while keeping the temperature at 20°—25°C.

At the end of the addition, butadiene bubbling is continued for a further 3 hours. The reaction mixture is then centrifuged to remove the catalyst, and is distilled on a rectification column, 5.5 g of butadiene monoepoxide (boiling point 65°C) being obtained.

Example 5
Allylglycidylether (AGE)

To 30 g of diallylether (DAE) (0.3 mol) in 25 cc of methanol plus 1 g of titanium-silicalite (as of Example 4), 9 cc of 60% $H_2O_2$ (0.2 mol) are added. The reaction mixture is kept stirred for 2 hours at 20°C. The reaction mixture is centrifuged to remove the catalyst, and the solvent and excess of DAE are distilled in vacuo at room temperature. As the residue, 17.5 g of 95%-pure AGE are obtained.

TABLE 1

| TS-1 | | | Silicalite a | | |
|---|---|---|---|---|---|
| 2θ, (CuK $\bar{\alpha}$) | Interplanar distance, d (Å) | Relative intensity (b) | 2θ, (CuK $\bar{\alpha}$) | Interplanar distance, d (Å) | Relative intensity (b) |
| 7.94 | 11.14 | vs | 7.94 | 11.14 | vs |
| 8.85 | 9.99 | s | 8.85 | 9.99 | s |
| 9.08 | 9.74 | m | 9.08 | 9.74 | m |
| 13.21 | 6.702 | w | 13.24 | 6.687 | w |
| 13.92 | 6.362 | mw | 13.95 | 6.348 | mw |
| 14.78 | 5.993 | mw | 14.78 | 5.993 | mw |
| 15.55 | 5.698 | w | 15.55 | 5.698 | w* |
| 15.90 | 5.574 | w | 15.90 | 5.574 | w |
| 17.65 | 5.025 | w | 17.65 | 5.025 | w |
| 17.81 | 4.980 | w | 17.83 | 4.975 | w |
| 20.37 | 4.360 | w | 20.39 | 4.355 | w |
| 20.85 | 4.260 | mw | 20.87 | 4.256 | mw |
| 23.07 | 3.855 | s | 23.08 | 3.853 | s |
| | | | 23.28 | 3.821 | ms |
| 23.29 | 3.819 | s | | | |
| | | | 23.37 | 3.806 | ms |
| | | | 23.71 | 3.753 | ms |
| 23.72 | 3.751 | s | | | |
| | | | 23.80 | 3.739 | ms |
| 23.92 | 3.720 | s | 23.94 | 3.717 | s |
| | | | 24.35 | 3.655 | mw |
| 24.41 | 3.646 | m | | | |
| | | | 24.60 | 3.619 | mw |
| | | | 25.84 | 3.448 | w |
| 25.87 | 3.444 | w | | | |
| | | | 25.97 | 3.431 | w |
| 26.87 | 3.318 | w* | 26.95 | 3.308 | w* |
| | | | 29.23 | 3.055 | w |

TABLE 1 (cont.)

| TS-1 | | | Silicalite a | | |
|---|---|---|---|---|---|
| 2θ, (CuK $\bar{\alpha}$) | Interplanar distance, d (Å) | Relative intensity (b) | 2θ, (CuK $\bar{\alpha}$) | Interplanar distance, d (Å) | Relative intensity (b) |
| 29.27 | 3.051 | mw | | | |
| | | | 29.45 | 3.033 | w |
| 29.90 | 2.988 | mw | 29.90 | 2.988 | mw |
| 30.34 | 2.946 | w | 30.25 | 2.954 | w |
| 45.00 | 2.014 | mw* | 45.05 | 2.012 | mw* |
| 45.49 | 1.994 | mw* | 45.60 | 1.989 | mw* |

(a) Prepared according to the modalities as disclosed in U.S. Patent 4,061,724; product calcined at 550°C.
(b) vs=very strong; s=strong; ms=medium-strong; m=medium; mw=medium-weak; w=weak; *=multiplet.

**Claims**

1. A process for the mono-epoxidation of compounds containing two olefinic double bonds separated by a number of carbon atoms, or of equivalent groups, lower than 8, with the exclusion of isoprene, which comprises contacting said compounds with hydrogen peroxide at a concentration of from 3 to 70% by weight, in the presence of a Si-Ti synthetic zeolite, wherein Ti and Si, in the form of their oxides $TiO_2$ and $SiO_2$ have a molar ratio in the zeolite corresponding to the formula

$$xTiO_2(1-x)SiO_2$$

wherein x is from 0.0001 to 0.04, possibly in the presence of at least one polar solvent, at such a temperature and under such a pressure as to keep the whole reaction mass in the liquid phase.

2. The process of claim 1 wherein x is from 0.0055 to 0.036.

3. The process of claim 1 wherein the polar solvent is selected from the group consisting of alcohols, ketones, ethers, glycols, glycol-ethers with a number of carbon atoms $\leq 6$.

4. The process of claim 3 wherein the alcohols are methanol and tert-butanol.

5. The process of claim 3 wherein the ketone is acetone.

6. The process of claim 3 wherein the ether is 2-methoxy-1-propanol.

7. The process of claim 1 wherein the temperature is from 0°C to 150°C, and the pressure is from 0.10 to 20.26 bar (from 0.1 to 20 abs. atm).

**Patentansprüche**

1. Verfahren zur Mono-Epoxidation von Verbindungen, die zwei olefinische Doppelbindungen, getrennt durch eine Anzahl von Kohlenstoffatomen oder von äquivalenten Gruppen, niedriger als 8, enthalten, mit Ausnahme von Isopren, dadurch gekennzeichnet, daß diese Verbindungen mit Wasserstoffperoxid bei einer Konzentration von 3 bis 70 Gew.-% in Anwesenheit eines Si-Ti synthetischen Zeoliths in Kontakt gebracht werden, wobei Ti und Si in Form ihrer Oxide $TiO_2$ und $SiO_2$ ein Molverhältnis in dem Zeolith entsprechend der Formel

$$xTiO_2(1-x)SiO_2$$

haben, wobei x von 0,0001 bis 0,04 ist, gegebenenfalls in Gegenwart von wenigstens einem polaren Lösungsmittel, bei einer solchen Temperatur und unter einem solchen Druck, daß die ganze Reaktionsmasse in flüssiger Phase gehalten wird.

2. Verfahren gemäß Anspruch 1, worin x von 0,0055 bis 0,036 beträgt.

3. Verfahren gemäß Anspruch 1, dadurch gekennzeichnet, daß das polare Lösungsmittel ausgewählt ist aus der Gruppe bestehend aus Alkoholen, Ketonen, Ethern, Glykolen, Glykolethern mit einer Kohlenstoffatomanzahl $\leq 6$.

4. Verfahren gemäß Anspruch 3, dadurch gekennzeichnet, daß die Alkohole Methanol und tert.-Butanol sind.

5. Verfahren gemäß Anspruch 3, dadurch gekennzeichnet, daß das Keton Aceton ist.

6. Verfahren gemäß Anspruch 3, dadurch gekennzeichnet, daß der Ether 2-Methoxy-1-propanol ist.

7. Verfahren gemäß Anspruch 1, dadurch gekennzeichnet, daß die Temperatur von 0°C bis 150°C und der Druck von 0,10 bis 20,26 bar (von 0,1 bis 20 abs. atm) beträgt.

**Revendications**

1. Procédé de monoépoxydation de composés contenant deux doubles liaisons oléfiniques séparées par un nombre d'atomes de carbone ou de groupes équivalents inférieur à 8, à l'exclusion de l'isoprène, qui comprend la mise en contact desdits composés avec du péroxyde d'hydrogène à une concentration de 3 à 70% en poids, en présence d'une zéolite synthétique Si-Ti dans laquelle Ti et Si, sous la forme de leurs oxydes $TiO_2$ et $SiO_2$, sont dans un rapport molaire correspondant à la formule:

$$xTiO_2(1\text{-}x)SiO_2$$

dans laquelle x a une valeur allant de 0,0001 à 0,04, éventuellement en présence d'au moins un solvant polaire, à une température et sous une pression telles que la masse réactionnelle globale soit maintenue à l'état de phase liquide.

2. Procédé de la revendication 1, dans lequel x a une valeur allant de 0,0055 à 0,036.

3. Procédé de la revendication 1 dans lequel le solvant polaire est choisi dans le groupe constitué par les alcools, les cétones, les éthers, les glycols, et les éthers de glycols ayant un nombre d'atomes de carbone inférieur ou égal à 6.

4. Procédé de la revendication 3 dans lequel les alcools sont le méthanol et le butanol tertiaire.

5. Procédé de la revendication 3 dans lequel la cétone est l'acétone.

6. Procédé de la revendication 3 dans lequel l'éther est le 2-méthoxy-1-propanol.

7. Procédé de la revendication 1 dans lequel la température est de 0°C à 150°C et la pression est de 0,10 à 20,26 bar (de 0,1 à 20 atmosphère absolue).

FIG. 1

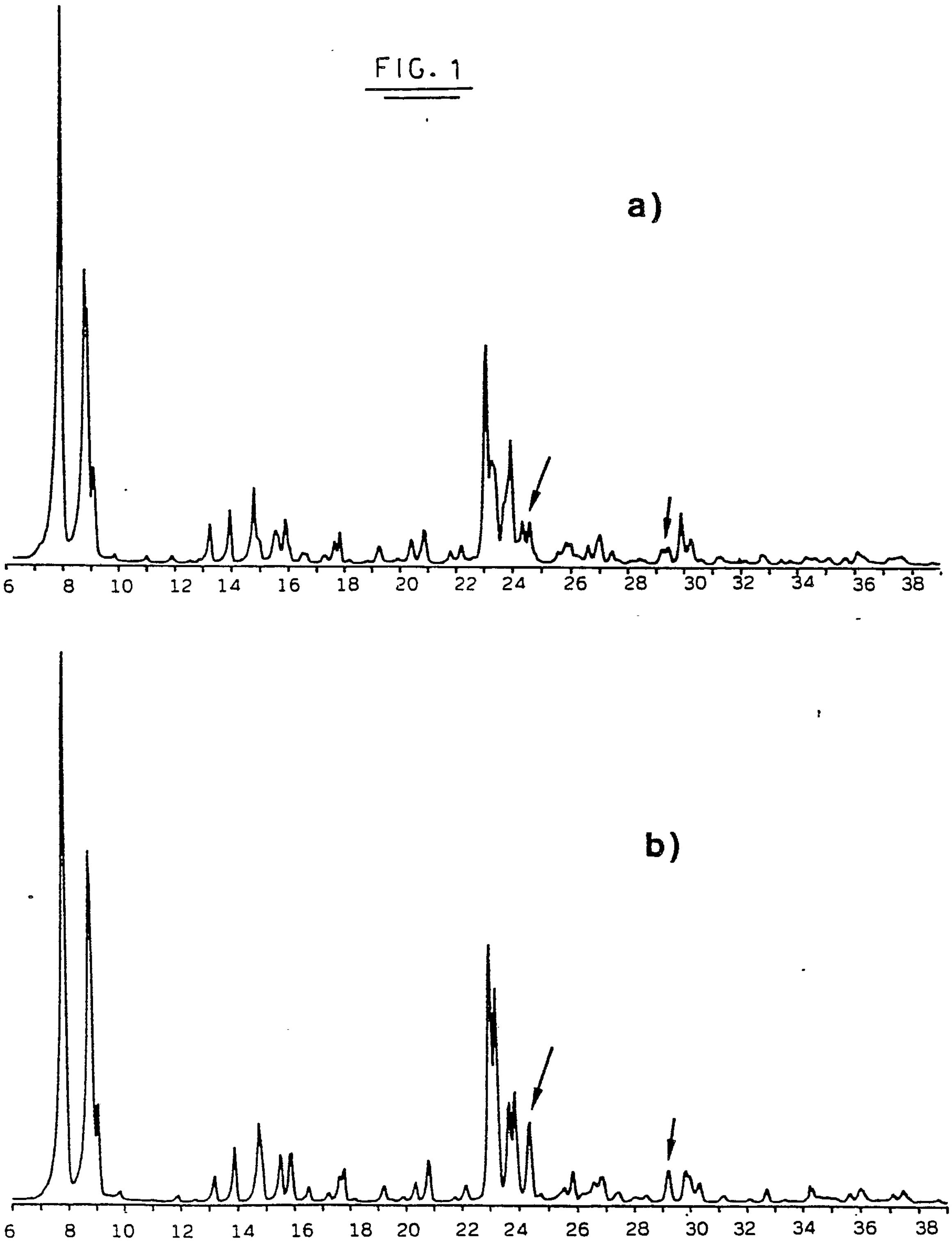
a)
6 8 10 12 14 16 18 20 22 24 26 28 30 32 34 36 38
b)
6 8 10 12 14 16 18 20 22 24 26 28 30 32 34 36 38

FIG. 2